# FASCICULE DE BREVET EUROPEEN

(11) **EP 3 174 512 B1**
(45) Date de publication et mention de la délivrance du brevet: **08.01.2020**
(21) Numéro de dépôt: 15752942.1
(22) Date de dépôt: 29.07.2015
(51) Int. Cl.: A61G 9/00, A61F 5/44, A61J 19/06

(54) **POCHE COMPORTANT UN CLAPET DE SÉCURITÉ**
BEUTEL MIT EINEM SICHERHEITSVENTIL
POUCH COMPRISING A SAFETY VALVE

(30) Priorité: 29.07.2014 FR 1457343
(43) Date de publication de la demande: 07.06.2017
(73) Titulaire: Swiss Safe Collect SA, 2000 Neuchâtel (CH)
(72) Inventeur: CAILLETEAU, Benoît, 1807 Blonay (CH)
(74) Mandataire: Cabinet Beau de Loménie
(86) Numéro de dépôt international: PCT/EP2015/067362
(87) Numéro de publication internationale: WO 2016/016296

(56) Documents cités:
- EP-A1- 0 117 016
- FR-A1- 2 995 210
- GB-A- 2 441 114

## Description

### Arrière-plan de l'invention

La présente invention concerne une poche comprenant une enveloppe souple ayant une ouverture à une extrémité, et un clapet de sécurité disposé à l'intérieur de l'enveloppe pour sensiblement empêcher le contenu de l'enveloppe de sortir par l'ouverture.

Des poches de ce type sont connues, par exemple, par les brevets européens n° 0 748 620 ou n° 0 847 742, et notamment par le brevet français n° 2 995 210. Elles sont utilisées pour recevoir des produits sous forme généralement liquide, notamment des déchets d'origine humaine ou animale tels que de l'urine ou des vomissures.

Ces poches sont couramment réalisées au moyen de feuilles minces de matière plastique (par exemple du polyéthylène) ou autres matériaux souples. Elles comportent un clapet de sécurité disposé à l'intérieur de l'enveloppe pour sensiblement empêcher le contenu de l'enveloppe de sortir par l'ouverture, par exemple lorsque la poche est accidentellement renversée.

Un tel clapet comporte au moins deux paires de feuilles imbriquées liées localement entre elles en une pluralité de points de liaison alignés parallèlement à et au voisinage d'une extrémité distale (rectiligne ou sensiblement rectiligne) des feuilles et régulièrement espacés. Par exemple, les feuilles sont de minces feuilles de matière plastique, et les points de liaison alignés sont réalisés par fusion locale de la matière plastique.

Avec cette disposition, un liquide piégé dans l'enveloppe ne peut pas, en principe, quitter l'espace compris entre les parois de l'enveloppe et la paire de feuilles extérieure du clapet. De plus, puisque le clapet comporte plusieurs paires de feuilles imbriquées, si une petite quantité de liquide vient à pénétrer entre la paire de feuilles extérieure et la paire de feuilles imbriquée dans celle-ci, le liquide tend à rester piégé entre ces deux paires de feuilles.

Après de nombreux essais portant sur l'agencement des points de liaison, l'inventeur a identifié le fait que l'espacement entre les points de liaison joue un rôle crucial dans le fonctionnement du clapet.

En effet, l'agencement des points de liaison doit être choisi de sorte à obtenir un bon compromis entre deux objectifs contraires : d'une part, faciliter le passage du liquide au moment de son introduction de la poche, et d'autre part, gêner ou empêcher le passage du liquide après qu'il a été piégé dans l'enveloppe.

L'inventeur s'est rendu compte que l'aptitude du clapet à gêner ou empêcher le passage du liquide piégé dans la poche dépend de l'écartement entre les points de liaison dans la direction parallèle à l'ouverture, c'est-à-dire dans la direction perpendiculaire à la direction d'introduction du liquide dans la poche.

Or, dans les poches du type cité plus haut, les points de liaison sont simplement alignés parallèlement à l'extrémité distale des feuilles, et l'écartement entre les points de liaison dans la direction perpendiculaire à la direction d'introduction du liquide est faible, afin de limiter les fuites du liquide piégé dans l'enveloppe.

Cette disposition présente des inconvénients.

En effet, le faible écartement entre les points de liaison tend à limiter le passage du liquide lorsque celui-ci est introduit dans la poche, ce qui peut nuire à une bonne utilisation de la poche.

De plus, lorsque le liquide contient des éléments solides (éléments solides dans des vomissures, caillots de sang dans de l'urine, etc.), les points de liaison peuvent gêner ou même empêcher le passage de ces éléments solides.

### Objet et résumé de l'invention

La présente invention vise à remédier à ces inconvénients.

L'invention vise à proposer une poche dont le clapet de sécurité permette un passage plus aisé d'éléments solides entre les points de liaison.

Ce but est atteint grâce à une poche comprenant une enveloppe souple ayant une ouverture à une extrémité et un clapet de sécurité disposé à l'intérieur de l'enveloppe pour sensiblement empêcher le contenu de l'enveloppe de sortir par l'ouverture, le clapet comprenant une première paire de feuilles, chaque feuille de la première paire de feuilles présentant une extrémité proximale fixée à une paroi de l'enveloppe au voisinage de l'ouverture et une extrémité distale opposée à l'extrémité proximale selon une direction longitudinale de l'enveloppe, une deuxième paire de feuilles, chaque feuille de la deuxième paire de feuilles s'étendant à l'intérieur de la première paire de feuilles, et présentant une extrémité proximale située au voisinage de l'ouverture et une extrémité distale plus proche de l'ouverture dans ladite direction longitudinale que l'extrémité distale des feuilles de la première paire de feuilles, et une troisième paire de feuilles, chaque feuille de la troisième paire de feuilles s'étendant à l'intérieur de la deuxième paire de feuilles, et présentant une extrémité proximale au voisinage de l'ouverture et une extrémité distale plus proche de l'ouverture dans ladite direction longitudinale que l'extrémité distale des feuilles de la deuxième paire de feuilles, dans lequel les feuilles de la première paire de feuilles et de la deuxième paire de feuilles sont liées localement entre elles, au voisinage de l'extrémité distale des feuilles de la deuxième paire de feuilles, en une pluralité de points de liaison primaires espacés les uns des autres et ne liant pas les feuilles de la troisième paire de feuilles aux feuilles de la première ou de la deuxième paires de feuilles, et les feuilles de la première, de la deuxième et de la troisième paires de feuilles sont liées localement entre elles en une pluralité de points de liaison secondaires espacés les uns des autres, et la distance entre les points de liaison primaires et l'extrémité distale des feuilles de la deuxième paire de feuilles varie le long d'une direction transversale de l'enveloppe.

Avec cette configuration, il est possible à la fois de conserver un faible écartement entre les points de liaison primaires dans la direction transversale (c'est-à-dire dans la direction perpendiculaire à la direction d'introduction dans la poche), ce qui gêne ou empêche le passage du liquide piégé dans la poche, et de ménager des espaces plus importants entre les points de liaison primaires, ce qui facilite le passage des éléments solides. Au sens de la présente description, l'«espace» ou la « distance » entre deux points de liaison voisins signifie la longueur du segment rectiligne entre ces deux points de liaison. Ainsi, la poche peut être utilisée de manière plus hygiénique lorsque celle-ci est utilisée pour recevoir un mélange généralement liquide comprenant des éléments solides, tel que des vomissures ou de l'urine contenant des calculs. De plus, lorsque la poche est utilisée pour recevoir une émulsion de deux liquides dont l'un est dense, par exemple de l'urine contenant du sang ou des caillots de sang, le passage du liquide le plus dense est facilité, ce qui permet un usage plus hygiénique de la poche.

Selon une possibilité, les points de liaison primaires comprennent un premier ensemble de points de liaison primaires alignés parallèlement à l'extrémité distale des feuilles de la deuxième paire de feuilles, et un second ensemble de points de liaison primaires plus éloignés de ladite extrémité distale que les points de liaison primaires du premier ensemble.

La fabrication de la poche est alors simplifiée, puisqu'il suffit de prévoir deux lignes de points de liaison primaires décalés l'un par rapport à l'autre et formant respectivement le premier et le deuxième ensemble, dont l'une est parallèle à l'extrémité distale des feuilles de la deuxième paire de feuilles.

De plus, par rapport à une configuration dans laquelle tous les points de liaison primaires sont alignés parallèlement au voisinage de l'extrémité distale des feuilles, on limite l'apparition de déformations ou de retraits de matière indésirables liés à la présence des points de liaison primaires et qui nuisent à l'étanchéité du clapet.

Selon une possibilité, les points de liaison primaires du second ensemble sont disposés en quinconce par rapport aux points de liaison primaires du premier ensemble.

De cette manière, on répartit uniformément les déformations du matériau des feuilles. L'étanchéité et la résistance du clapet sont donc améliorées.

Selon l'invention, le clapet comprend une troisième paire de feuilles, chaque feuille de la troisième paire de feuilles s'étendant à l'intérieur de la deuxième paire de feuilles, et présentant une extrémité proximale au voisinage de l'ouverture et une extrémité distale plus proche de l'ouverture dans ladite direction longitudinale que l'extrémité distale des feuilles de la deuxième paire de feuilles,

Du fait de la présence d'une troisième paire de feuilles et des points de liaison secondaires, l'étanchéité du clapet est encore améliorée, car cette troisième paire de feuilles et ces points de liaison secondaires constituent une gêne supplémentaire au passage du liquide piégé dans l'enveloppe.

Selon l'invention, les points de liaison primaires lient localement entre elles les feuilles de la première et la deuxième paires de feuilles, et les points de liaison secondaires lient localement entre elles les feuilles de la première, de la deuxième et de la troisième paire de feuilles.

Ainsi, l'étanchéité du clapet est encore améliorée.

Selon une possibilité, la distance entre les points de liaison secondaires et l'extrémité distale des feuilles de l'une quelconque des paires de feuilles varie le long de ladite direction transversale.

Selon une possibilité, les points de liaison secondaires comprennent un premier ensemble de points de liaison secondaires alignés parallèlement à l'extrémité distale des feuilles de l'une quelconque des paires de feuilles, et un second ensemble de points de liaison secondaires plus éloignés de ladite extrémité distale que les points de liaison secondaires du premier ensemble.

Selon une possibilité, les points de liaison secondaires du second ensemble sont disposés en quinconce par rapport aux points de liaison secondaires du premier ensemble.

Les avantages pré-cités, concernant les points de liaison primaires, se retrouvent ainsi également pour les points de liaison secondaires.

Selon une possibilité, l'écartement entre deux points de liaison primaires voisins et l'écartement entre deux points de liaison secondaires voisins augmentent dans le même sens le long de ladite direction transversale.

La poche selon l'invention, qui vient d'être définie selon ses différents modes de réalisation, permet d'obtenir les effets suivants.

Puisqu'on ménage des espaces importants entre les points de liaison primaires, le passage d'éléments solides est grandement facilité. D'autre part, lorsque la poche est utilisée pour recevoir une émulsion de deux liquides dont l'un est dense, par exemple de l'urine contenant du sang ou des caillots de sang, le passage du liquide le plus dense est facilité. Ceci permet un usage plus hygiénique de la poche.

De plus, lorsqu'on utilise la poche pour recevoir une émulsion de deux liquides, par exemple de l'urine contenant du sang ou des caillots de sang, la séparation des liquides est facilitée. En particulier, lorsque les liquides ont une densité sensiblement différente, lorsqu'on incline la poche vers l'horizontale (c'est-à-dire de sorte que sa direction transversale soit inclinée d'environ 30° à 60° par rapport à la verticale) et de manière que les points de liaison les plus espacés soient situés en bas, les liquides sont séparés par gravité.

### Brève description des dessins

L'invention sera bien comprise et ses avantages apparaîtront mieux, à la lecture de la description détaillée qui suit de plusieurs modes de réalisations, représentés à titre d'exemples non limitatifs. La description se réfère aux dessins annexés sur lesquels :
- la figure 1 est une vue d'une poche selon un premier mode de réalisation, dans sa configuration aplatie ;
- la figure 2 est un agrandissement de la zone II de la figure 1, montrant mieux le clapet ;
- la figure 3 est une vue d'une poche selon un deuxième mode de réalisation, dans sa configuration aplatie ;
- la figure 4 est un agrandissement de la zone IV de la figure 3, montrant mieux le clapet ;
- la figure 5 est un agrandissement de la même zone, montrant le clapet selon un troisième mode de réalisation.

### Description détaillée de l'invention

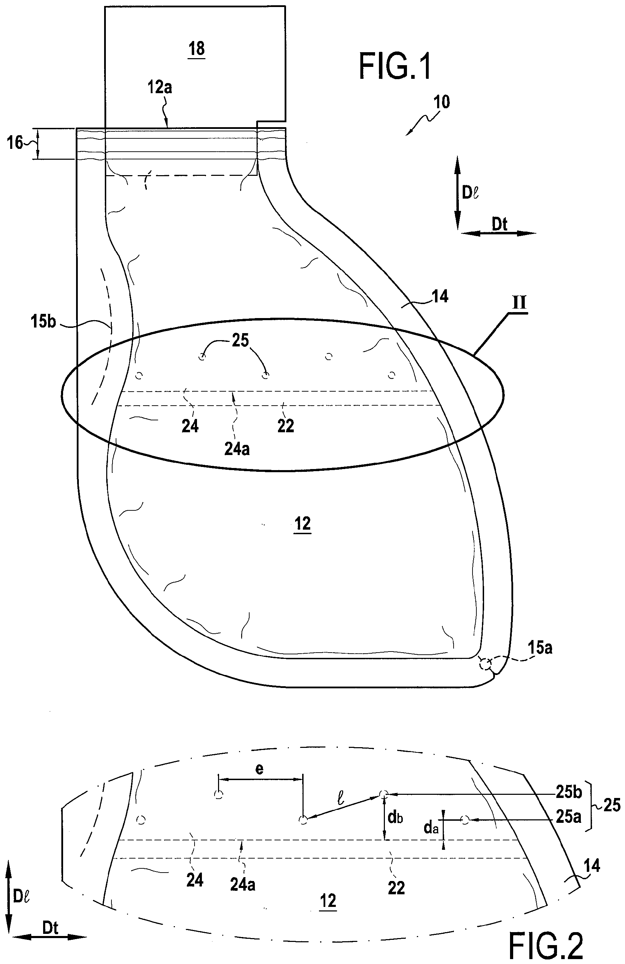

La poche 10 représentée sur la figure 1 comprend une enveloppe souple 12. En particulier, cette enveloppe est réalisée à partir de deux feuilles minces de matière plastique découpées de manière convenable et soudées l'une à l'autre sur la quasi-totalité du contour, par une ligne de soudure 14. L'enveloppe 12 présente une ouverture 12a ménagée par une interruption de la ligne de soudure 14, pour permettre à l'espace intérieur de l'enveloppe de communiquer avec l'extérieur.

Dans l'exemple représenté, cette ouverture est ménagée à l'extrémité libre d'une portion de col 16 que présente l'enveloppe, dans laquelle la largeur de cette enveloppe est réduite.

L'enveloppe 12 peut passer d'une configuration aplatie pour un stockage aisé, à une configuration d'utilisation dans laquelle elle peut recevoir des déchets.

Par exemple, les feuilles qui forment l'enveloppe sont en matière plastique souple, comme du polyéthylène.

La poche comprend un manchon de renfort 18 qui est fixé en travers de l'ouverture 12a et fait saillie à l'extérieur de l'enveloppe.

Le manchon 18 peut avoir toute forme convenable, pourvu qu'il puisse être fixé à l'enveloppe 12 au travers de son ouverture 12a, et qu'il puisse passer d'une configuration aplatie à une configuration d'utilisation dans laquelle il ménage un canal pour l'introduction des déchets. Des manchons adaptés pour cela sont décrits, par exemple, dans le brevet européen n° 0 847 742, ou dans la publication de demande de brevet français n° 2 995 210.

On constate également sur la figure 1 que, dans une région opposée à l'ouverture, la ligne de soudure 14 présente une ligne d'affaiblissement 15a, réalisée par exemple à l'aide de micro perçages en pointillées. Cet affaiblissement s'étend seulement sur la ligne de soudure, sans atteindre l'espace interne de l'enveloppe. Lorsque la poche est pleine de déchets, ceux-ci peuvent ainsi être vidés en déchirant l'enveloppe selon la ligne d'affaiblissement 15a, avec un effort suffisant pour que la déchirure ainsi créée s'étende jusqu'à l'espace interne de l'enveloppe.

Par ailleurs, sur l'un de ces côtés voisins de l'ouverture, la ligne de soudure 14 présente une autre ligne d'affaiblissement 15b, pouvant être également réalisée par des perçages discontinus. Cette ligne est à distance de l'espace interne de l'enveloppe et elle est orientée de telle sorte que sa déchirure ne peut pas se propager jusqu'à l'intérieur de l'enveloppe. Ainsi, lorsque la poche est pleine et que l'on souhaite la stocker avant de la vider, la ligne 15b peut être déchirée pour ménager une fente permettant d'accrocher la poche à un crochet ou analogue.

Comme on le voit sur la figure 1, la poche présente un clapet de sécurité constitué de plusieurs feuilles internes disposées contre chacune des parois de la poche.

Dans l'exemple représenté, le clapet comporte une première paire de feuilles 22 qui s'étend, dans la direction longitudinale Df de l'enveloppe 12, depuis l'ouverture 12a jusqu'à une région médiane de la poche, et une deuxième paire de feuilles 24 qui s'étend à l'intérieur de la paire de feuilles 22, les feuilles de la deuxième paire de feuilles 24 étant plus courtes que les feuilles de la première paire de feuilles 22.

Lorsque l'enveloppe est dans sa configuration d'utilisation, le liquide est introduit sensiblement parallèlement à la direction longitudinale Dℓ de l'enveloppe 12.

Les feuilles s'étendent sur toute la largeur de la poche dans la région où elles se trouvent et sont soudées entre elles et avec les parois de l'enveloppe par la ligne de soudure 14.

En particulier, l'extrémité proximale (proche de l'ouverture 12a) des feuilles des première et deuxième paires de feuilles 22 et 24 est fixée à une paroi de l'enveloppe 12 au voisinage de l'ouverture 12a, par la ligne de soudure 14.

Comme cela est représenté sur la figure 1, les feuilles de la première paire de feuilles 22 et les feuilles de la deuxième paire de feuilles 24 sont liées localement entre elles en une pluralité de points de liaison primaires 25, au voisinage de l'extrémité distale (sensiblement rectiligne) 24a des feuilles de la paire de feuilles 24.

Les feuilles des première et deuxième paires de feuilles 22, 24 peuvent être en matière plastique (par exemple, de minces feuilles de polyéthylène). Dans ce cas, les points de liaison primaires 25 sont réalisés par fusion locale de la matière plastique.

L'invention s'intéresse tout particulièrement à la disposition des points de liaison primaires 25.

Ainsi, comme cela est mieux visible sur la figure 2, les points de liaison primaires 25 sont agencés de telle sorte que la distance entre les points de liaison primaires 25 et l'extrémité distale 24a des feuilles de la paire de feuilles 24 varie le long de la direction transversale Dt de l'enveloppe.

Au sens de la présente description, par « la distance entre les points de liaison 25 et l'extrémité distale 24a », il faut entendre « la plus courte distance entre les points de liaison 25 et l'extrémité distale 24a ».

Dans l'exemple représenté, les points de liaison primaires 25 comprennent un premier ensemble de points de liaison primaires 25a alignés parallèlement à l'extrémité distale 24a des feuilles de la paire de feuilles 24 et situés à une distance dₐ de l'extrémité distale 24a, et un second ensemble de points de liaison primaires 25b situés à une distance d_{b} de l'extrémité distale 24a, la distance d_{b} étant plus grande que la distance dₐ.

Avec cette configuration, l'écartement entre les points de liaison primaires 25 parallèlement à la direction transversale Dt (c'est-à-dire sensiblement perpendiculairement à la direction d'introduction du liquide), représenté par e sur la figure 2, peut être conservé suffisamment faible pour garantir une bonne étanchéité du clapet, tout en ménageant une distance ℓ entre un point de liaison primaire 25a et un point de liaison primaire 25b voisin suffisamment importante pour laisser passer des éléments solides. En d'autres termes, cette configuration permet de conserver l'étanchéité du clapet tout en ménageant des espaces suffisants entre les points de liaison 25a et les points de liaison 25b pour laisser passer des éléments solides.

En outre, puisque les points de liaison primaires 25 comprennent un second ensemble de points de liaison 25b plus éloignés de l'extrémité distale 24a que le premier ensemble, l'extrémité distale est moins déformée par la présence des points de liaison et conserve une forme sensiblement rectiligne. L'étanchéité du clapet est donc améliorée.

Dans l'exemple représenté, les points de liaison 25b sont alignés parallèlement à l'extrémité distale 24a. Cependant, on peut envisager d'autres configurations où les points de liaison 25b ne sont pas alignés parallèlement à l'extrémité distale 24a, ou encore ne sont pas alignés.

Dans l'exemple représenté, les points de liaison 25b sont disposés en quinconce par rapport aux points de liaison 25a. C'est-à-dire que les points de liaison 25b sont alignés parallèlement à l'extrémité distale 24a, et décalés par rapport aux points de liaison 25a. Dans ce cas, l'écartement parallèlement à la direction transversale Dt entre deux points de liaison 25a voisins quelconques, et l'écartement entre deux points de liaison 25b quelconques parallèlement à la direction transversale Dt, sont identiques.

Avec cette configuration, les déformations du matériau des feuilles des première et deuxième paires de feuilles 22 et 24 sont uniformément réparties. L'étanchéité et la résistance du clapet sont donc améliorées.

Toutefois, ces écartements peuvent aussi être différents, sans pour autant sortir de la portée de l'invention.

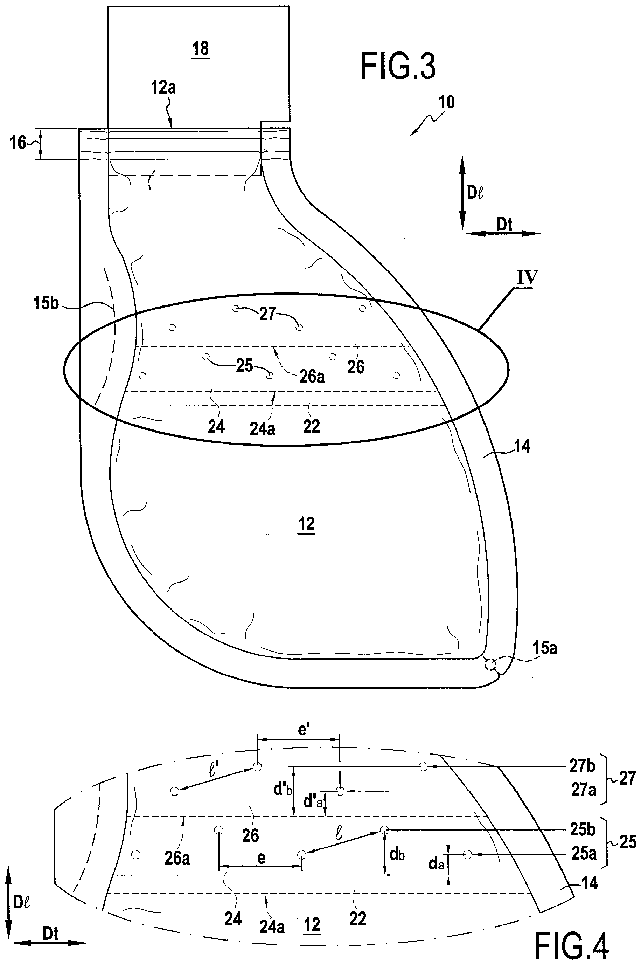

Dans un deuxième mode de réalisation, représenté sur la figure 3, le clapet comporte également une troisième paire de feuilles 26 s'étendant sur toute la largeur de la poche dans la région où elles se trouvent, et soudées avec les parois de l'enveloppe par la ligne de soudure 14. En particulier, l'extrémité proximale (proche de l'ouverture 12a) des feuilles de la paire de feuilles 26 est fixée à une paroi de l'enveloppe au voisinage de l'ouverture 12a, par la ligne de soudure 14.

La troisième paire de feuilles 26 s'étend à l'intérieur de la paire de feuilles 24, les feuilles de la troisième paire de feuilles 26 étant plus courtes que les feuilles de la paire de feuilles 24. Les feuilles des première et deuxième paires de feuilles 22, 24 sont liées localement entre elles par les points de liaison primaires 25 comme cela a été décrit auparavant.

Les première, deuxième et troisième paires de feuilles 22, 24 et 26 sont liées localement entre elles en une pluralité de points de liaison secondaires 27 au voisinage de l'extrémité distale 26a de la paire de feuilles 26.

Les feuilles de la troisième paire de feuilles 26 peuvent être en matière plastique (par exemple, de minces feuilles de polyéthylène). Dans ce cas, les points de liaison secondaires 27 sont réalisés par fusion locale de la matière plastique.

Les feuilles de la troisième paire de feuilles 26 s'étendent dans un volume intérieur de l'enveloppe 12 défini par les feuilles de la deuxième paire de feuilles 24.

On comprend de ce qui précède que les points de liaison primaires 25 lient localement entre elles les feuilles des première et deuxième paires de feuilles 22 et 24 seulement, et que les points de liaison secondaires 27 lient localement entre elles les feuilles des première, deuxième et troisième paires de feuilles 22, 24 et 26. Les points de liaison primaires 25 ne lient pas les feuilles de la troisième paire de feuilles 26 aux feuilles de la première paire de feuilles 22 ou de la deuxième paire de feuilles 24.Les points de liaison primaires 25 rendent les feuilles des première et deuxième paires de feuilles 22 et 24 solidaires les unes des autres, les feuilles de la troisième paire de feuilles 26 n'étant pas solidaires des feuilles des première et deuxième paires de feuilles 22 et 24 par le biais des points de liaison de primaires 25. En outre, comme la distance entre les points de liaison primaires 25 et l'extrémité distale 24a des feuilles de la paire de feuilles 24 varie le long de la direction transversale Dt de l'enveloppe comme cela a été décrit ci-dessus, le passage d'éléments solides à travers le clapet est facilité.

Les points de liaison secondaires 27 rendent les feuilles des première, deuxième et troisième paires de feuilles 22, 24 et 26 solidaires les unes des autres.

Comme cela est mieux visible sur la figure 4, les points de liaison secondaires 27 sont agencés de telle sorte que la distance entre les points de liaison secondaires 27 et l'extrémité distale 26a des feuilles de la paire de feuilles 26 varie le long de la direction transversale Dt de l'enveloppe 12.

Au sens de la présente description, par « la distance entre les points de liaison 27 et l'extrémité distale 26a », il faut entendre « la plus courte distance entre les points de liaison 27 et l'extrémité distale 26a ».

Les points de liaison secondaires 27 comprennent un premier ensemble de points de liaison secondaires 27a alignés parallèlement à l'extrémité distale des feuilles de l'une quelconque des paires de feuilles, et un second ensemble de points de liaison secondaires 27b plus éloignés de l'extrémité distale que les points de liaison secondaires 27a. Par exemple, comme cela est représenté sur la figure 4, les points de liaison secondaires 27a sont situés à une distance d'ₐ de l'extrémité distale 26a des feuilles de la paire de feuilles 26, et les points de liaison 27b sont situés à une distance d'_{b} de l'extrémité distale 26a, la distance d'_{b} étant plus grande que la distance d'ₐ.

Avec cette configuration, l'écartement entre les points de liaison secondaires 27 parallèlement à la direction transversale Dt (c'est-à-dire sensiblement perpendiculairement à la direction d'introduction du liquide), représenté par e' sur la figure 4, peut être conservé suffisamment faible pour garantir une bonne étanchéité du clapet, tout en ménageant une distance ℓ' entre un point de liaison secondaire 27a et un point de liaison secondaire 27b voisin suffisamment importante pour laisser passer des éléments solides.

Ainsi, les éléments solides peuvent passer entre les points de liaison secondaires 27a et 27b (écartement e' et distance ℓ'), puis entre les points de liaison primaires 25a et 25b (écartement e et distance ℓ), comme cela a été décrit ci-dessus.

En d'autres termes, cette configuration permet d'améliorer encore l'étanchéité du clapet tout en ménageant des espaces suffisants entre les points de liaison pour laisser passer des éléments solides.

Selon une possibilité, et comme cela est représenté sur la figure 4, l'écartement e' peut être sensiblement égal à l'écartement e.

Cependant, il peut aussi être intéressant de choisir un écartement e' différent de l'écartement e.

Par exemple, on peut choisir un écartement e' légèrement supérieur à l'écartement e. Ainsi, au moment de l'introduction du liquide, les points de liaison secondaires 27 étant davantage espacés, le liquide n'est pas refoulé au niveau des points de liaison secondaires 27, et peut progresser jusqu'aux points de liaison primaires 25 situés en-dessous, puis jusqu'à l'intérieur de l'enveloppe 12. D'autre part, après que le liquide a été introduit dans la poche, son passage en direction de l'extérieur de l'enveloppe 12 est d'abord gêné par les points de liaison primaires 25, qui sont moins espacés et forment donc une gêne plus importante à la fuite du liquide.

Bien entendu, pour que les points de liaison secondaires 27 continuent à contribuer à l'étanchéité du clapet, l'écartement e' ne doit pas être trop important ou trop supérieur à l'écartement e.

On peut aussi choisir un écartement e' légèrement inférieur à l'écartement e. Ainsi, après que le liquide a été introduit dans la poche, son passage en direction de l'extérieur de l'enveloppe 12 est gêné en premier lieu par les points de liaison primaires 25, et en second lieu par la troisième paire de feuilles 26 et les points de liaison secondaires 27 qui, étant moins espacés, forment une gêne plus importante à la fuite du liquide.

En outre, puisque les points de liaison secondaires 27 comprennent un second ensemble de points de liaison 27b plus éloignés de l'extrémité distale 26a que le premier ensemble, l'extrémité distale est moins déformée par la présence des points de liaison et conserve une forme sensiblement rectiligne. L'étanchéité du clapet est donc améliorée.

Dans l'exemple représenté, les points de liaison 27b sont alignés parallèlement à l'extrémité distale 26a. Cependant, on peut envisager d'autres configurations où les points de liaison 27b ne sont pas alignés parallèlement à l'extrémité distale 26a, ou encore ne sont pas alignés.

Dans l'exemple représenté, les points de liaison 27b sont disposés en quinconce par rapport aux points de liaison 27a. C'est-à-dire que les points de liaison 27b sont alignés parallèlement à l'extrémité distale 26a, et décalés par rapport aux points de liaison 27a. Dans ce cas, l'écartement entre deux points de liaison 27a voisins quelconques parallèlement à la direction transversale Dt, et l'écartement entre deux points de liaison 27b quelconques parallèlement à la direction transversale Dt, sont identiques.

Avec cette configuration, les déformations du matériau des feuilles des première, deuxième et troisième paires de feuilles 22, 24 et 26 sont uniformément réparties. L'étanchéité et la résistance du clapet sont donc améliorées.

Toutefois, ces écartements peuvent aussi être différents, sans pour autant sortir de la portée de l'invention.

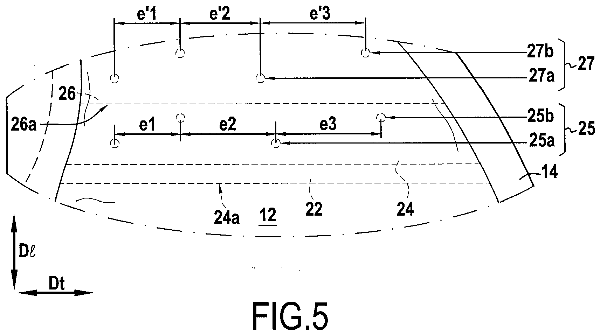

Dans un troisième mode de réalisation, représenté sur la figure 5, les points de liaison 25 et 27 sont disposés de telle sorte que l'écartement entre deux points de liaison primaires 25 voisins successifs augmente dans un sens donné le long de la direction transversale Dt, depuis le bord d' l'enveloppe 12 formé par la ligne de soudure 14 à proximité de la ligne d'affaiblissement 15b et jusqu'à l'autre bord de l'enveloppe 12 formé par la ligne de soudure 14, et que l'écartement entre deux points de liaison secondaires 27 voisins successifs augmente dans le même sens le long de la direction transversale Dt.

Plus précisément, comme cela est représenté sur la figure 5, les écartements successifs e₁, e₂, e₃, ... entre des points de liaison primaires 25 voisins successifs sont tels que : e₁ ≤ e₂ ≤ e₃ ≤ .... De même, les écartements successifs e'₁, e'₂, e'₃, ... entre des points de liaison secondaires 27 voisins successifs sont tels que : e'₁ ≤ e'₂ ≤ e'₃ ≤ ....

Avec cette configuration, lorsqu'on incline la poche 10 vers l'horizontale (c'est-à-dire de sorte que la direction transversale Dt fasse un angle d'environ 30° à 60° par rapport à la verticale) et de manière que les points de liaison 25, 27 les plus espacés soient situés en bas, et qu'on utilise la poche 10 pour recevoir un mélange de liquides de densités différentes, les liquides sont séparés par gravité, et la séparation des liquides est facilitée.

Dans une variante, on peut prévoir que seuls les points de liaison primaires 25, ou seuls les points de liaison secondaires 27, soient disposés de sorte que l'écartement entre deux points de liaison voisins successifs augmente dans un sens le long de la direction transversale Dt, sans pour autant sortir de la portée de l'invention.

Quoique la présente invention ait été décrite en se référant à des exemples de réalisation spécifiques, il est évident que des différentes modifications et changements peuvent être effectués sur ces exemples sans sortir de la portée générale de l'invention telle que définie par les revendications. En outre, des caractéristiques individuelles des différents modes de réalisation évoqués peuvent être combinées dans des modes de réalisation additionnels. Par conséquent, la description et les dessins doivent être considérés dans un sens illustratif plutôt que restrictif.

## Revendications

1. Poche (10) comprenant :
une enveloppe souple (12) ayant une ouverture (12a) à une extrémité ;
un clapet de sécurité disposé à l'intérieur de l'enveloppe (12) pour sensiblement empêcher le contenu de l'enveloppe (12) de sortir par l'ouverture (12a), ledit clapet comprenant :
une première paire de feuilles (22), chaque feuille de la première paire de feuilles présentant une extrémité proximale fixée à une paroi de l'enveloppe (12) au voisinage de l'ouverture (12a) et une extrémité distale opposée à l'extrémité proximale selon une direction longitudinale (Dℓ) de l'enveloppe,
une deuxième paire de feuilles (24), chaque feuille de la deuxième paire de feuilles s'étendant à l'intérieur de la première paire de feuilles (22), et présentant une extrémité proximale située au voisinage de l'ouverture (12a) et une extrémité distale (24a) plus proche de l'ouverture (12a) dans ladite direction longitudinale (Dℓ) que l'extrémité distale des feuilles de la première paire de feuilles (22), et
une troisième paire de feuilles (26), chaque feuille de la troisième paire de feuilles s'étendant à l'intérieur de la deuxième paire de feuilles (24), et présentant une extrémité proximale au voisinage de l'ouverture (12a) et une extrémité distale (26a) plus proche de l'ouverture (12a) dans ladite direction longitudinale (Dℓ) que l'extrémité distale (24a) des feuilles de la deuxième paire de feuilles (24),
la poche étant **caractérisée en ce que** :
les feuilles de la première paire de feuilles (22) et de la deuxième paire de feuilles (24) sont liées localement entre elles, au voisinage de l'extrémité distale (24a) des feuilles de la deuxième paire de feuilles (24), en une pluralité de points de liaison primaires (25) espacés les uns des autres et ne liant pas les feuilles de la troisième paire de feuilles (26) aux feuilles de la première (22) ou de la deuxième paires de feuilles (24) ;
les feuilles de la première (22), de la deuxième (24) et de la troisième paires de feuilles (26) sont liées localement entre elles en une pluralité de points de liaison secondaires (27) espacés les uns des autres ;
la distance entre les points de liaison primaires (25) et l'extrémité distale (24a) des feuilles de la deuxième paire de feuilles (24) varie le long d'une direction transversale (Dt) de l'enveloppe (12).

2. Poche selon la revendication 1, **caractérisée en ce que** les points de liaison primaires comprennent :
un premier ensemble de points de liaison primaires (25a) alignés parallèlement à l'extrémité distale (24a) des feuilles de la deuxième paire de feuilles (24) ; et
un second ensemble de points de liaison primaires (25b) plus éloignés de ladite extrémité distale (24a) que les points de liaison primaires du premier ensemble (25a).

3. Poche selon la revendication 2, **caractérisée en ce que** les points de liaison primaires du second ensemble (25b) sont disposés en quinconce par rapport aux points de liaison primaires du premier ensemble (25a).

4. Poche selon l'une quelconque des revendications 1 à 3, **caractérisée en ce que** la distance entre les points de liaison secondaires (27) et l'extrémité distale des feuilles de l'une quelconque des paires de feuilles varie le long de ladite direction transversale (Dt).

5. Poche selon la revendication 4, **caractérisée en ce que** les points de liaison secondaires (27) comprennent :
un premier ensemble de points de liaison secondaires (27a) alignés parallèlement à l'extrémité distale des feuilles de l'une quelconque des paires de feuilles; et
un second ensemble de points de liaison secondaires (27b) plus éloignés de ladite extrémité distale que les points de liaison secondaires du premier ensemble (27a).

6. Poche selon la revendication 5, **caractérisée en ce que** les points de liaison secondaires du second ensemble (27b) sont disposés en quinconce par rapport aux points de liaison secondaires du premier ensemble (27a).

7. Poche selon l'une quelconque des revendications 1 à 6, **caractérisée en ce que** l'écartement (e₁, e₂, e₃) entre deux points de liaison primaires (25) voisins et l'écartement (e'₁, e'₂, e'₃) entre deux points de liaison secondaires (27) voisins augmentent dans le même sens le long de ladite direction transversale (Dt).

## Patentansprüche

1. Beutel (10), umfassend:
eine flexible Hülle (12), die eine Öffnung (12a) an einem Ende aufweist,
ein Sicherheitsventil, das innerhalb der Hülle (12) angeordnet ist, um im Wesentlichen zu verhindern, dass der Inhalt der Hülle (12) durch die Öffnung (12a) austritt, wobei das Ventil umfasst:
ein erstes Paar von Folien (22), wobei jede Folie des ersten Folienpaars ein proximales Ende, das an einer Wand der Hülle (12) in der Nähe der Öffnung (12a) befestigt ist, sowie ein distales Ende aufweist, das dem proximalen Ende in einer Längsrichtung (Dℓ) der Hülle gegenüberliegt,
ein zweites Paar von Folien (24), wobei sich jede Folie des zweiten Folienpaars innerhalb des ersten Folienpaars (22) erstreckt und ein proximales Ende, das in der Nähe der Öffnung (12a) angeordnet ist, sowie ein distales Ende (24a) aufweist, das in der Längsrichtung (Dℓ) näher an der Öffnung (12a) gelegen ist als das distale Ende der Folien des ersten Folienpaars (22), und
ein drittes Paar von Folien (26), wobei sich jede Folie des dritten Folienpaars innerhalb des zweiten Folienpaars (24) erstreckt und ein proximales Ende in der Nähe der Öffnung (12a) sowie ein distales Ende (26a) aufweist, das in der Längsrichtung (Dℓ) näher an der Öffnung (12a) gelegen ist als das distale Ende (24a) der Folien des zweiten Folienpaars (24),
wobei der Beutel **dadurch gekennzeichnet ist, dass**:
die Folien des ersten Folienpaars (22) und des zweiten Folienpaars (24) in der Nähe des distalen Endes (24a) der Folien des zweiten Folienpaars (24), an einer Vielzahl von primären Verbindungsstellen (25), die voneinander beabstandet sind und die Folien des dritten Folienpaars (26) nicht mit den Folien des ersten Folienpaars (22) oder des zweiten Folienpaars (24) verbinden, lokal untereinander verbunden sind,
die Folien des ersten Folienpaars (22), des zweiten Folienpaars (24) und des dritten Folienpaars (26) an einer Vielzahl von sekundären Verbindungsstellen (27), welche voneinander beabstandet sind, lokal untereinander verbunden sind,
der Abstand zwischen den primären Verbindungsstellen (25) und dem distalen Ende (24a) der Folien des zweiten Folienpaars (24) entlang einer Querrichtung (Dt) der Hülle (12) variiert.

2. Beutel nach Anspruch 1, **dadurch gekennzeichnet, dass** die primären Verbindungsstellen umfassen:
eine erste Anordnung von primären Verbindungsstellen (25a), die parallel zu dem distalen Ende (24a) der Folien des zweiten Folienpaars (24) ausgerichtet sind, und
eine zweite Anordnung von primären Verbindungsstellen (25b), die von dem distalen Ende (24a) weiter entfernt sind als die primären Verbindungsstellen der ersten Anordnung (25a).

3. Beutel nach Anspruch 2, **dadurch gekennzeichnet, dass** die primären Verbindungsstellen der zweiten Anordnung (25b) gegenüber den primären Verbindungsstellen der ersten Anordnung (25a) versetzt angeordnet sind.

4. Beutel nach einem der Ansprüche 1 bis 3, **dadurch gekennzeichnet, dass** der Abstand zwischen den sekundären Verbindungsstellen (27) und dem distalen Ende der Folien von einem der Folienpaare entlang der Querrichtung (Dt) variiert.

5. Beutel nach Anspruch 4, **dadurch gekennzeichnet, dass** die sekundären Verbindungsstellen (27) umfassen:
eine erste Anordnung von sekundären Verbindungsstellen (27a), die parallel zu dem distalen Ende der Folien von einem der Folienpaare ausgerichtet sind, und
eine zweite Anordnung von sekundären Verbindungsstellen (27b), die von dem distalen Ende weiter entfernt sind als die sekundären Verbindungsstellen der ersten Anordnung (27a).

6. Beutel nach Anspruch 5, **dadurch gekennzeichnet, dass** die sekundären Verbindungsstellen der zweiten Anordnung (27b) gegenüber den sekundären Verbindungsstellen der ersten Anordnung (27a) versetzt angeordnet sind.

7. Beutel nach einem der Ansprüche 1 bis 6, **dadurch gekennzeichnet, dass** der Abstand (e₁, e₂, e₃) zwischen zwei benachbarten primären Verbindungsstellen (25) und der Abstand (e'₁, e'₂, e'₃) zwischen zwei benachbarten sekundären Verbindungsstellen (27) in der gleichen Richtung entlang der Querrichtung (Dt) zunehmen.

## Claims

1. A pouch (10) comprising:
• a flexible bag (12) having an opening (12a) at one end;
• a safety valve arranged inside the bag (12) to substantially prevent the content of the bag (12) leaving via the opening (12a), said valve comprising:
• a first pair of sheets (22), each sheet of the first pair of sheets having a proximal end in the vicinity of the opening (12a) that is secured to a wall of the bag (12) and a distal end opposite the proximal end along a longitudinal axis (Dℓ) of the bag;
• a second pair of sheets (24), each sheet in the second pair of sheets extending inside the first pair of sheets (22) and having a proximal end situated in the vicinity of the opening (12a), and a distal end (24a) closer to the opening (12a) along said longitudinal axis (Dℓ) than the distal end of the sheets of the first pair of sheets (22); and
• a third pair of sheets (26), each sheet of the third pair of sheets extending inside the second pair of sheets (24) and having a proximal end in the vicinity of the opening (12a), and a distal end (26a) closer to the opening (12a) along said longitudinal axis (Dℓ) than the distal end (24a) of the sheets of the second pair of sheets (24);
the pouch being **characterized in that**:
• the sheets of the first pair of sheets (22) and of the second pair of sheets (24) are locally bonded together in the vicinity of the distal end (24a) of the sheets of the second pair of sheets (24), at a plurality of primary bonding points (25) that are spaced apart from one another and that do not bond the sheets of the third pair of sheets (26) with the sheets of the first (22) or the second (24) pairs of sheets; and
• the sheets of the first (22), second (24), and third (26) pairs of sheets are locally bonded together by a plurality of secondary bonding points (27) that are spaced apart from one another;
the distance between the primary bonding points (25) and the distal end (24a) of the sheets of the second pair of sheets (24) varies along a transverse axis (Dt) of the bag (12).

2. A pouch according to claim 1, **characterized in that** the primary bonding points comprise:
• a first set of primary bonding points (25a) aligned parallel to the distal end (24a) of the sheets of the second pairs of sheets (24); and
• a second set of primary bonding points (25b) further away from said distal end (24a) than the primary bonding points of the first set (25a).

3. A pouch according to claim 2, **characterized in that** the primary bonding points of the second set (25b) are arranged in a staggered configuration relative to the primary bonding points of the first set (25a).

4. A pouch according to any one of claims 1 to 3, **characterized in that** the distance between the secondary bonding points (27) and the distal end of the sheets of any of the pairs of sheets varies along said transverse axis (Dt).

5. A pouch according to claim 4, **characterized in that** the secondary bonding points (27) comprise:
• a first set of secondary bonding points (27a) aligned parallel to the distal end of the sheets of any one of the pairs of sheets; and
• a second set of secondary bonding points (27b) further away from said distal end than the secondary bonding points of the first set (27a).

6. A pouch according to claim 5, **characterized in that** the secondary bonding points of the second set (27b) are arranged in a staggered configuration relative to the secondary bonding points of the first set (27a).

7. A pouch according to any one of claims 1 to 6, **characterized in that** the spacing (e₁, e₂, e₃) between two neighboring primary bonding points (25) and the spacing (e'₁, e'₂, e'₃) between two neighboring secondary bonding points (27) increases in the same direction along said transverse axis (Dt).
